# EUROPÄISCHE PATENTSCHRIFT

(11) **EP 1 411 037 B1**
(45) Veröffentlichungstag und Bekanntmachung des Hinweises auf die Patenterteilung: **14.12.2011**
(21) Anmeldenummer: 03022295.4
(22) Anmeldetag: 02.10.2003
(51) Int. Cl.: C05D 9/02, C07C 229/24, C07C 229/76

(54) **Verfahren zur Herstellung chelatisierter Pflanzenspurennährstoffe**
Process for obtaining plant micronutrients
Procédé d'obtention des substances nutritives chélatées pur végétaux

(30) Priorität: 15.10.2002 DE 10248022
(43) Veröffentlichungstag der Anmeldung: 21.04.2004
(73) Patentinhaber: LANXESS Deutschland GmbH, 51369 Leverkusen (DE); ADOB Przedsiebiorstwo Produkcyjno-Consultingowe Limited partnership company, 61-028 Poznan (PL)
(72) Erfinder: Mitschker, Alfred, Dr., 51519 Odenthal (DE); Moritz, Ralf-Johann, Dr., 41469 Neuss (DE); Nawrocki, Adam, 61306 Poznan (PL)

(56) Entgegenhaltungen:
- EP-A- 1 359 140
- PATENT ABSTRACTS OF JAPAN vol. 1997, no. 09, 30. September 1997 (1997-09-30) & JP 09 136807 A (NITTO CHEM IND CO LTD;TOMOE KAGAKU KOGYO KK), 27. Mai 1997 (1997-05-27) & DATABASE WPI Derwent Publications Ltd., London, GB; AN 1997-337018

## Beschreibung

Die vorliegende Erfindung betrifft ein Verfahren zur Herstellung chelatisierter Pflanzenspurennährstoffe mit dem Reaktionsprodukt aus dem Tetranatriumsalz der N-(1,2-Dicarboxylethyl)-D,L-asparaginsäure und deren Mischungen mit Metallionen aus der Gruppe der anorganischen oder organischen Zink-, Mangan-, Eisen(II), Eisen(III)- oder Kupfer(II)-Verbindungen.

Um ein ordentliches Pflanzenwachstum zu gewährleisten, werden Spurennährstoffe wie Eisen, Kupfer, Zink und Mangan ausgebracht. Spurennährstoffe werden in chelatisierter Form besser von den Pflanzen aufgenommen, und ein Mangel, der zu geringerer Ernte führt, wird ausgeglichen.

Die Anwendung von Metallionen in chelatisierter Form, die mit entsprechenden Komplexbildnern mit hohen Stabilitätskonstanten hergestellt werden, ist bereits aus dem Stand der Technik bekannt. Chelatisierte Metallionen gewährleisten die rasche Aufnahme und Translokation innerhalb der Pflanze unter verschiedenen Wachstumsbedingungen, wie Boden-pH, Wechselwirkung zwischen Bodenbestandteilen, Klimabedingungen, Bicarbonatgehalt, Redoxpotenzial und anderen Parametern.

Chelatisierte Eisen(II)-, Eisen(III)-, Mangan-, Kupfer- und Zinkionen werden in Form einzelner Spurenelemente oder in Form von Mischungen sowie als Zusatzmittel für NPK-Voll- oder -Mischdünger (NPK = Stickstoff-Phosphor-Kali) verwendet

JP-A Hei 9-136807 beschreibt als Pflanzendünger einen Ammoniumkomplex des Eiseniminodisuccinats. EP1359140A1 beschreibt die Herstellung und Verwendung von Iminodibernsteinsäureammoniumsalzen.

Aus DE-A 3 517 102 ist zum Beispiel ein Flüssigdünger mit chelatisiertem Eisen(III), Mangan, Kupfer, Zink oder Kobalt in Form von Nitraten mit einem pH von 4 bis 8 und einer Konzentration von 40,3 % bis zu 62,7 % der Trockenmasse bekannt. Im genannten Stand der Technik wurden die Chelatisierungsmittel Nitrilotriessigsäure (NTA), Ethylendiamintetraessigsäure (EDTA), Diethylentriaminopentaessigsäure (DTPA), N-Hydroxyethyl-ethylendiamintriessigsäure (HEEDTA), Ethylendiamin-di-(o-hydroxyphenyl-essigsäure) (EDDHA) getrennt oder in Kombinationen mit ihren Natrium-, Kalium- und Ammoniumsalzen in einem Molverhältnis von Metall zu Chelatisierungsmittel von mindestens 0,1:1,0 bis 5:1, vorzugsweise 0,8:1 bis 2,5:1,0, verwendet.

Die meisten der im Stand der Technik genannten synthetisch hergestellten Chelatisierungsmittel sind nicht biologisch abbaubar und reichern sich folglich in Böden und Wasserläufen an.

DE19713911A1 beschreibt die Herstellung und ein Verfahren zur Herstellung von Iminodibernsteinsäuren.

In DE-A 1 0219 037 wird ein Verfahren zur Herstellung von Iminodibemsteinsäureammoniummetallsalzen sowie deren möglicher Einsatz als Spurennährstoffdünger beschrieben. Es finden sich dort jedoch keine Hinweise, dass die zwei-, drei- oder vierfachen Alkalimetall- oder Alkalimetallammoniummischsalze der N-(1,2-Dicarboxyethyl)-D,L-asparaginsäure oder deren Mischungen die Erfordernisse eines biologisch abbaubaren Spurennährstoffdüngers besonders gut erfüllen.

Es bestand daher die Aufgabe, ein Verfahren zur Herstellung von Pflanzennährstoffen in chelatisierter Form den Pflanzen zur Verfügung zu stellen, die Spurennährstoffe in chelatisierter Form binden und diese den Pflanzen in ausreichender Menge zur Verfügung stellen, wobei gleichzeitig die Chelatisierungsmittel möglichst vollständig biologisch abbaubar sind.

Die Aufgabe wird gelöst durch ein Verfahren gemäß Anspruch 1.
Die chelatisierten Pflanzennährstoffe sollen dabei zu mindestens 70,0 % in einem Zeitraum von 28 Tagen gemäß OECD-Richtlinien No 301 E biologisch abgebaut sein. Sie eignen sich deshalb hervorragend zur Düngung von Pflanzen, insbesondere den Nutzpflanzen.

Die Erfindung betrifft ein Verfahren, bei dem als Verbindung der Formel (A) 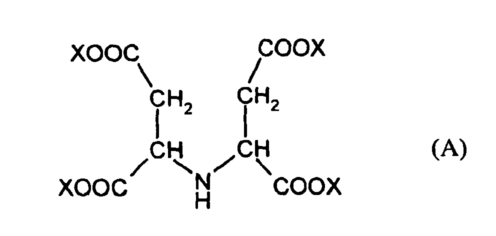 worin
4 X für Natrium stehen, eingesetzt wird.

Verbindungen (B) sind erfindungsgemäß Carbonate, Chloride, Sulfate, Oxide, Hydroxide, Acetate und Nitrate der Metalle Eisen III, Eisen II, Mangan, Kupfer, Zink.

Erfindungsgemäß bevorzugt wird ein Molverhältnis zwischen dem Chelatisierungsmittel (A) und dem Metallion (B) im Bereich von 1,3-0,8 zu 1,0-0,9 eingestellt.

Die nach dem erfindungsgemäßen Verfahren herzustellenden chelatisierten Spurennährstoffe werden sowohl in flüssiger als auch fester Form hergestellt und enthalten gegebenenfalls üblicherweise verwendete Zusatzstoffe.

Die nach dem erfindungsgemäßen Verfahren herzustellenden Flüssigprodukte enthalten 1,0 bis 6,0 Gew.-% des Spurennährstoffs, wobei das bevorzugte Molverhältnis zu dem Chelatisierungsmittel 0,95 bis 1,0 beträgt.

Die nach dem erfindungsgemäßen Verfahren herzustellenden Festprodukte enthalten 5,0 bis 14,0 Gew.-% des Spurennährstoffs, wobei das bevorzugte Molverhältnis zu dem Chelatisierungsmittel 0,95 bis 1,0 beträgt.

Weiterhin können die nach dem erfindungsgemäßen Verfahren herzustellenden chelatisierten Spurennährstoffe andere in der Landwirtschaft, im Gartenbau oder in der Hydroponik verwendete Spurennährstoffe wie Calcium, Magnesium, Bor, Molybdän oder Kobalt enthalten.

Es wurde gefunden, dass die nach dem erfindungsgemäßen Verfahren herzustellenden chelatisierten Spurennährstoffe als einzelne Chelate oder deren Mischung mit anderen bekannten komplexbildenden Verbindungen der Reihe Aminopolycarboxylverbindungen, Polyaminocarboxylverbindungen, Poly- und Bicarboxylverbindungen, Hydroxypolycarboxylverbindungen, Hydroxypolyaminocarboxylverbindungen sowie gegebenenfalls als Bestandteil von NPK-Voll- und -Mischdüngern ausgebracht werden können, was ihr Anwendungsgebiet und ihre Wirksamkeit erhöht.

Bevorzugte Volldünger sind Stickstoffdünger wie beispielsweise UAN-Lösung 30,0 %, Phosphordünger wie beispielsweise MAP oder DAP oder Kalidünger wie beispielsweise MOP, SOP, KNO₃ oder Mischungen der verschiedenen Volldünger untereinander.

Erfindungsgemäß bevorzugt kann der nach dem erfindungsgemäßen Verfahren herzustellende chelatisierte Pflanzenspurennährstoff noch Netzmittel oder Haftmittel enthalten. Bevorzugte Netzmittel oder Haftmittel sind Cycocel^{®}, Ligninsulfonate oder Gluconate.

Die vorliegende Erfindung betrifft ferner bevorzugt ein Verfahren zur Herstellung der Fertigprodukte in fester oder flüssiger Form.

Das Chelatisieren wird dabei durch Reaktion des Komplexbildners (A) mit einer Iminogruppe mit Polycarboxylgruppen und einer anorganischen Verbindung (B) eines Chlorids, Nitrats, Acetats, Sulfats der mehrwertigen Metallionen, des Eisens, Mangans, Kupfers oder Zinks oder durch Reaktion des Komplexbildners (A) mit einer anorganischen Verbindung (C) eines Hydroxids, Carbonats oder Oxids derselben mehrwertigen Metallionen mit Zugabe von anorganischen oder organischen Säuren durchgeführt. Bevorzugte Säuren im Sinne der vorliegenden Erfindung sind Salzsäure, Schwefelsäure, Salpetersäure oder Essigsäure.

Um die erhaltenen Produkte in die feste Form zu überführen, werden die flüssigen Spurennährstoffdünger in einer Sprühtrocknungsanlage getrocknet Hierzu werden die flüssigen Produkte vorteilhafterweise zunächst filtriert und dann unter einem Druck von 15 - 60 bar, vorzugsweise 35 - 45 bar, mit entsprechenden Düsen in einen Sprühturm gesprüht. Die Eingangstemperatur des Sprühturmes beträgt hierbei 100 - 300°C, bevorzugt 120 - 250°C und die Ausgangstemperatur liegt bei 50 - 150°C, bevorzugt 70 - 120°C. Man erhält ein annähernd staubfreies Microgranulat mit einer Korngröße von 50 - 400 µm, bevorzugt 80 - 300 µm. Es hat sich als vorteilhaft erwiesen, das anfallende Mircogranulat auf ca. 30°C zu kühlen und mit einem Antihaftmittel zu konditionieren. Hierzu können beispielsweise Produkte der Hostapur^{®}-Produktserie verwendet werden.

Zu den Ausbringungsmöglichkeiten der erfindungsgemäßen Flüssigprodukte oder Festprodukte zählen Blattsprays, Ausbringung auf den Boden, Hydroponik sowie Bewässerungsdüngung.

### Beispiele

### Beispiel 1 (erfindungsgemäß)

23 ml einer 34 %igen N-(1,2-Dicarboxyethyl)-D,L-asparaginsäure-tetranatriumsalzlösung wurden bei 40°C unter Rühren mit 20 ml einer 18,0 %igen Zinkchloridlösung versetzt.

Nach 1-stündigem Umsetzen nach Zugabe von 0,3 % Ligninsulfonat als Haftmittel erhielt man eine durchsichtige Lösung, die lagerstabil war.

Der Zn-Gehalt betrug 3,74 Gew.-%.

### Beispiel 2 (erfindungsgemäß)

19,6 ml einer 34 %igen N-(1,2-Dicarboxyethyl)-D,L-asparaginsäure-tetranatriumsalzlösung wurden tropfenweise bei 60°C unter Rühren mit 20 ml einer 20 %igen Mangan(II)-nitratlösung versetzt.

Nach 2-stündigem Umsetzen bei 60°C wurde 0,5 % Cycocel^{®} als Netzmittel zugegeben, wodurch man eine orangefarbene durchsichtige Lösung erhielt, die lagerstabil war.

Der Mn-Gehalt betrug 2,9 Gew.-% (w/w).

### Beispiel 3 (nicht erfindungsgemäß)

12,9 ml einer 47,0 %igen N-(1,2-Dicarboxyethyl)-D,L-asparaginsäure-ammoniumdikaliumsalzlösung wurden bei 40°C unter Rühren mit 20 ml einer 27,0 %igen Kupfer(II)-nitratlösung versetzt.

Nach 1-stündigem Umsetzen bei 40°C wurden 0,5 % Cycocel^{®} als Netzmittel zu der Lösung gegeben, wodurch man zu einer durchsichtigen blauen Lösung gelangte, die lagerstabil war.

Der Cu-Gehalt betrug 3,8 Gew.-%.

### Beispiel 4 (erfindungsgemäß)

20 ml einer 12,0 %igen Eisen(III)-nitratlösung wurden bei 40°C unter Rühren mit 11,5 ml einer 34,0 %igen N-(1,2-Dicarboxyethyl)-D,L-asparaginsäure-tetranatriumsalzlösung versetzt.

Nach 2-stündigem Umsetzen unter Lichtausschluss bei 40°C wurden 0,5 % Cycocel^{®} als Netzmittel und 0,5 % Ligninsulfonat als Haftmittel zugegeben, wodurch man zu einer dunkelgrünen durchsichtigen Lösung gelangte, die lagerstabil war.

Der Fe(II)-Gehalt betrug 2,22 Gew.-%.

### Beispiel 5 (erfindungsgemäß)

20 ml einer 12,0 %igen Eisen(III)-nitratlösung wurden bei 60°C unter Rühren mit 11,5 ml einer 34,0 %igen N-(1,2-Dicarboxyethyl)-D,L-asparaginsäure-tetranatriumsalzlösung versetzt.

Nach 1-stündigem Umsetzen wurden 0,5 % Oxidationsmittel, 0,5 % Cycocel^{®} als Netzmittel und 0,5 % Gluconat als Haftmittel zugegeben, und es wurde noch 1 Stunde lang gerührt.

Die zum Schluss erhaltene Lösung war eine durchsichtige dunkelrote Flüssigkeit, die lagerstabil war.

Der Fe(III)-Gehalt betrug 2,2 Gew.-%.

### Beispiel 6 (erfindungsgemäß)

393,5 ml einer 34,0 %igen N-(1,2-Dicarboxyethyl)-D,L-asparaginsäure-tetranatriumsalzlösung wurden bei 60°C unter Rühren mit 45 ml einer 20,0 %igen Zinknitratlösung, 33,7 ml einer 27,0 %igen Kupfer(II)-nitratlösung, 310,5 ml einer 12,0 %igen Eisen(III)-nitratlösung, 133,8 ml einer 20,0 %igen Mangannitratlösung, 13,7 g Borsäure und 60,8 g Magnesiumnitrat versetzt.

Nach 2-stündigem Umsetzen bei 60°C erhielt man eine durchsichtige dunkelgrüne Lösung, die lagerstabil war.

| | | |
|---|---|---|
| Die Lösung enthielt: | Zn | - 0,3 % |
| | Cu | - 0,3 % |
| | Fe | - 1,1 % |
| | Mn | - 0,8 % |
| | B | - 0,2 % |
| | MgO | - 0,8 % |
| Alle Angaben in % bedeuten Gew.-%. | | |

### Beispiel 7 (erfindungsgemäß)

23 ml einer 34,0 %igen N-(1,2-Dicarboxyethyl)-D,L-asparaginsäure-tetranatriumsalzlösung und 2,39 g ZnO (79,4 % ZnO) wurden bei 40°C mit 7,2 g einer 55,0 %igen Salpetersäure versetzt.

Nach zweistündiger Reaktionszeit wurde die Lösung filtriert und eine lagerstabile transparente Flüssigkeit erhalten. Der Zink-Gehalt lag bei 4,65 Gew.-%.

## Patentansprüche

1. Verfahren zur Herstellung chelatisierter Pflanzenspurennährstoffe **dadurch gekennzeichnet, dass** das Tetranatriumsalz der N-(1,2-Dicarboxyethyl)-D,L-asparaginsäure als Verbindung (A) mit einer anorganischen Verbindung (B) eines Chlorids, Nitrats, Acetats, Sulfats der mehrwertigen Metallionen des Eisens, Mangans, Kupfers oder Zink zur Reaktion gebracht wird oder diese durch Reaktion des Komplextbildners der Verbindung (A) mit einer anorganischen Verbindung (C) eines Hydroxids, Carbonats oder Oxids derselben mehrwertigen Metallionen mit Zugabe von anorganischen oder organischen Säuren entstehen.

2. Verfahren gemäß Anspruch 1, **dadurch gekennzeichnet, dass** als Säuren Salzsäure, Schwefelsäure, Salpetersäure oder Essigsäure eingesetzt werden.

3. Verfahren gemäß der Ansprüche 1 oder 2, **dadurch gekennzeichnet, dass** das Molverhältnis zwischen der Verbindung (A) und der Verbindung (B) im Bereich von 1,3:0,8 bis 1,0:0,9 liegt.

4. Verfahren gemäß der Ansprüche 1 bis 3, **dadurch gekennzeichnet, dass** das Reaktionsprodukt in flüssiger Form vorliegt und 1,0 bis 6,0 Gew.-% der Metallionen enthält.

5. Verfahren gemäß der Ansprüche 1 bis 4, **dadurch gekennzeichnet, dass** zur Überführung in eine feste Form die erhaltenen Produkte in einer Sprühtrocknungsanlage getrocknet werden.

## Claims

1. Process for the preparation of chelated plant micronutrients, **characterized in that** the tetrasodium salt of N-(1,2-dicarboxyethyl)-D,L-aspartic acid as compound (A) is reacted with an inorganic compound (B) of a chloride, nitrate, acetate, sulphate of the polyvalent metal ions of iron, manganese, copper or zinc or **in that** they are generated by reacting the complexing agent of the compound (A) with an inorganic compound (C) of a hydroxide, carbonate or oxide of the same polyvalent metal ions with addition of inorganic or organic acids.

2. Process according to Claim 1, **characterized in that** the acids employed are hydrochloric acid, sulphuric acid, nitric acid or acetic acid.

3. Process according to Claim 1 or 2, **characterized in that** the molar ratio between compound (A) and compound (B) is in the range of from 1.3:0.8 to 1.0:0.9.

4. Process according to Claims 1 to 3, **characterized in that** the reaction product is present in liquid form and comprises 1.0 to 6.0% by weight of the metal ions.

5. Process according to Claims 1 to 4, **characterized in that** the products obtained are dried in a spray-drying apparatus so that they are converted into a solid form.

## Revendications

1. Procédé de préparation d'oligo-éléments chélatés pour les plantes, **caractérisé en ce qu'**on fait réagir le sel tétrasodique de l'acide N-(1,2-dicarboxyéthyl)-D,L-asparagique en tant que composé (A) avec un composé inorganique (B) d'un chlorure, nitrate, acétate, sulfate des ions métalliques polyvalents du fer, du manganèse, du cuivre ou du zinc, ou que ces derniers sont obtenus par réaction du complexant du composé (A) avec un composé inorganique (C) d'un hydroxyde, d'un carbonate ou d'un oxyde de ces mêmes ions métalliques polyvalents, avec addition d'acides inorganiques ou organiques.

2. Procédé selon la revendication 1, **caractérisé en ce qu'**on utilise en tant qu'acides l'acide chlorhydrique, l'acide sulfurique, l'acide nitrique ou l'acide acétique.

3. Procédé selon les revendications 1 ou 2, **caractérisé en ce que** le rapport en moles entre le composé (A) et le composé (B) est compris dans la plage de 1,3:0,8 à 1,0:0,9.

4. Procédé selon les revendications 1 à 3, **caractérisé en ce que** le produit de la réaction se présente sous forme liquide et contient 1,0 à 6,0 % en poids des ions métalliques.

5. Procédé selon les revendications 1 à 4, **caractérisé en ce que**, pour une conversion en une forme solide, on soumet les produits obtenus à un séchage dans une installation de séchage par atomisation.
